# EUROPEAN PATENT SPECIFICATION

(11) **EP 3 922 936 B1**
(45) Date of publication and mention of the grant of the patent: **27.09.2023**
(21) Application number: 21167680.4
(22) Date of filing: 09.04.2021
(51) Int. Cl.: F25D 27/00, A61L 2/20

(54) **CONTROL PROCEDURE FOR THE SANITIZATION OF ICE-MAKING APPARATUS AND RELATED APPARATUS**
KONTROLLVERFAHREN FÜR DIE DESINFIZIERUNG VON EISHERSTELLUNGSGERÄTEN UND ZUGEHÖRIGEN GERÄTEN
PROCÉDURE DE CONTRÔLE POUR L'ASSAINISSEMENT DES APPAREILS DE FABRICATION DE GLACE ET APPAREILS CONNEXÉS

(30) Priority: 08.06.2020 IT 202000013546
(43) Date of publication of application: 15.12.2021
(73) Proprietor: CASTEL MAC S.R.L., 31033 Castelfranco Veneto (TV) (IT)
(72) Inventor: SPINETTI, Alberto Italo, 31033 CASTELFRANCO VENETO (TV) (IT)
(74) Representative: Burchielli, Riccardo

(56) References cited:
- EP-A1- 2 100 624
- EP-A1- 2 674 031
- EP-A2- 2 407 727
- WO-A1-2013/096808
- WO-A2-2008/101364
- US-A1- 2005 089 458
- US-A1- 2007 196 244
- US-A1- 2009 142 225
- US-A1- 2009 183 523
- US-A1- 2010 266 445
- US-A1- 2012 031 118
- US-A1- 2019 056 123

## Description

This invention relates to a control procedure for the sanitization of an ice-making apparatus and a related apparatus.

Nowadays, an ice-making apparatus allows ice to be produced in different quantities and shapes using a cooling circuit and an evaporator at a temperature of around -15°.

In particular, the water is transformed into ice during the so-called "cold" phase by coming into contact with the evaporator and then settling on its surface; the ice thus formed is periodically removed from the evaporator by defrosting with hot gas, i.e. by mechanical removal.

Such apparatus can be further equipped with an ice deposit which, when a certain level of saturation is reached, automatically stops making ice, which then resumes when the ice deposit is emptied.

To manage the operation of such apparatus, there are electronic devices, possibly combined with electromechanical components, which can be combined with a series of pumps to circulate and convey water to the evaporator for the formation of the ice.

On the basis of what has been said, such apparatus has a continuous production of ice until the machine is manually switched OFF or put in standby mode, or alternatively as already mentioned, until the sensor located in the deposit detects the presence of ice above a certain threshold.

In addition, at present this apparatus can also be fitted with sanitizing devices, such as ozone generators or UVC lamps that are independent but are not integrated with the logic of the machines, and therefore are not coordinated with the operation of the machines themselves; this determines the fact that these sanitizing devices generally remain activated at all times.

It is necessary for an ice-making apparatus to have sanitizing devices because although such apparatus uses drinking water, it is subject to the growth of organisms, since even drinking water contains a small concentration of bacteria when it enters the apparatus. This small concentration has the opportunity to take root on surfaces and grow in the machine.

As just mentioned, two types of sanitizing devices are currently used: UVC lamps or LEDs and ozone generators; these devices can be of various shapes, power and effectiveness, depending on the volume to be sanitized and the type of apparatus on which they are installed.

The two types of sanitizing devices have different limitations and merits; the most important differentiation is the ability to diffuse the sanitizing agent into the surrounding environment or the apparatus in which they are located.

Being in gaseous form, ozone is able to permeate a circumscribed volume almost in its entirety, having no limits or obstacles, while although UV lamps or UVC LEDs have greater germicidal efficacy, they are limited by the fact that by relying on radiation, they cannot always reach all spaces with the same intensity, especially if they are far away or in the shade compared to the cone of ultraviolet light that they can guarantee. In detail, UVC disinfection uses strong short-wave radiation (250-280 nm) to inactivate microorganisms by destroying their DNA, leaving them unable to perform their vital cellular functions. Conventional UV systems use mercury vapour lamps and although they are very effective, there are limitations to their use; integrating them into existing systems is often complicated as they are bulky and contain delicate materials such as glass.

Additionally, the operational management of the lamps comprises periodic replacement, significant energy consumption and the use and disposal of hazardous substances such as mercury.

The complete disinfection of the water in an ice-making apparatus with a UVC-LED sanitizing device is more effective than simple UV lamps because it prevents slime or biofilm (growth of organic pathogens), while also disinfecting the surrounding areas.

Lastly, the solutions that use ozone in gaseous form to sanitize the apparatus in question usually operate continuously, becoming potentially harmful or annoying from an olfactory point of view for the end user who may come into contact with this gas, as the machines are generally not airtight; furthermore, this continuous operation does not allow integrated actions to be coordinated with sanitizing devices that normally require discontinuous operation, such as germicidal UV lamps.

In particular, an ice-making apparatus having the technical characteristics described in the preamble of the appended claim 1 is known for example from patent documents US2005/089458A1, EP2100624A1 and US2010/266445A1 .As can easily be deduced for the reasons just given, the current solutions for the sanitization of an ice-making apparatus are disadvantageous, since they propose only one type of operation, regardless of the working conditions, frequency of maintenance, climatic conditions and the actual presence of contaminants and/or parasites, and they do not allow sanitization to be modulated according to the different working conditions of the apparatus or the user's needs.

This also means that the design of the sanitizing devices is often over-sized to ensure their effectiveness in all models and situations.

This is an inconvenience precisely because nowadays it is essential, also in view of the problems linked to the pandemic phenomena due to viruses, to ensure that an ice-making apparatus ensures total sanitization of its components and that it is adapted and modulated according to specific needs. The main aim of this invention is to provide a control procedure for the sanitization of an ice-making apparatus and a related apparatus such as to overcome the above limitations of the known art.

Another aim of this invention is to provide a control procedure for the sanitization of an ice-making apparatus such as to limit bacterial growth and the activity of any viruses, as well as the growth of moulds or parasitic organisms of plant or animal origin.

A further aim of this invention is to provide a control procedure for the sanitization of an ice-making apparatus and related apparatus, which enables ice of a higher hygienic quality to be obtained than what is possible today, with a lower percentage of biological agents, such as by way of example, viruses and bacteria.

A further aim of this invention is to provide a control procedure for the sanitization of an ice-making apparatus and related apparatus such that the need for cleaning and maintenance is reduced and the good condition of the apparatus is advantageously ensured.

A further aim of this invention is to provide a control procedure for the sanitization of an ice-making apparatus and related apparatus in such a way that the action of the sanitizing agents can be advantageously programmed, thus avoiding in this case, the release of an increased quantity of ozone into the air, especially when personnel or customers are in the vicinity, and also limiting access to the machines and their productions.

A further aim of this invention is to provide a control procedure for the sanitization of an ice-making apparatus with which different activation times of the sanitizing action can be decided.

Last but not least, the aim of this invention is to provide a control procedure for the sanitization of an ice-making apparatus such as to be adaptable to the use of different sanitization techniques, with different layouts for the positioning of the sanitizing devices, so as not to propose a fixed and rigid solution, rather one that is adaptable to all circumstances over time.

These and other aims are achieved by a control procedure for the sanitization of an ice-making apparatus according to attached claim 1, and by a related control method according to claim 5.

Further detailed technical features of the invention are contained in the dependent claims.

This invention will now be described by way of non-limiting example according to some of its preferred embodiments, with the aid of the attached figures, wherein:
- Figure 1a shows a block diagram relating to a first type of control procedure for the sanitization of an ice-making apparatus according to this invention;
- Figure 1b shows a block diagram relating to a second type of control procedure for the sanitization of an ice-making apparatus, the object of this invention;
- Figure 1c shows a block diagram of a third type of control procedure, the object of this invention;
- Figure 1d shows a block diagram of a fourth type of control procedure, the object of this invention;
- Figure 2a schematically shows the operation of the first type of sanitization control procedure in Figure 1a, according to the invention;
- Figure 2b schematically shows the operation of the second type of sanitization control procedure in Figure 1b, according to the invention;
- Figure 2c schematically shows the operation of the third type of sanitization control procedure in Figure 1c, according to the invention;
- Figure 2d schematically shows the operation of the fourth type of sanitization control procedure in Figure 1d, according to the invention;
- Figure 3a shows a perspective and rear view of a first embodiment of the ice-making apparatus, the object of this invention, having a first type of sanitizing device;
- Figure 3b shows a perspective and front view of the apparatus in Figure 3a, the object of this invention;
- Figure 4a shows a rear perspective view of the apparatus in Figure 3a, connected to a second type of sanitizing device;
- Figure 4b shows a perspective and front view of the apparatus in Figure 4a, the object of this invention;
- Figure 5 shows a perspective view of an ice containment apparatus, the object of this invention, connected to the second type of sanitizing device;
- Figure 6a shows a perspective and front view of a third embodiment of the ice-making apparatus, the object of this invention, having the first type of sanitizing device;
- Figure 6b shows a further perspective and front view of a third embodiment of the ice-making apparatus in Figure 6a, the object of this invention, having the first type of sanitizing device acting on the scoop for removing ice from the deposit;
- Figure 7a shows a perspective view of the ice-making apparatus in Figure 6a, having the second type of sanitizing device;
- Figure 7b is a further perspective view of the apparatus in Figure 7a, the object of this invention;
- Figure 8a is a perspective view of a fourth embodiment of the ice-making apparatus, the object of this invention, having the first type of sanitizing device;
- Figure 8b is a further perspective view of the apparatus in Figure 8a, the object of this invention;
- Figure 8c shows the ice-making apparatus in Figure 8b, the object of this invention, having the first type of sanitizing device acting on the scoop for removing ice from the deposit;
- Figure 9a is a perspective view of the ice-making apparatus in Figure 7a, the object of this invention, having the second type of sanitizing device;
- Figure 9b is a further perspective view of the apparatus in Figure 9a, the object of this invention.

With reference to the figures mentioned, the control procedure for the sanitization of an ice-making apparatus 10 advantageously makes it possible to manage the intervention times of the sanitizing devices, regardless of their type. Such procedure can be advantageously applied to any ice-making apparatus and aims to activate the sanitizing devices, thus ensuring greater wholesomeness of the ice produced.

This procedure allows the following different modes of operation to be advantageously implemented.

### A) Continuous manual activation of the sanitizing devices until subsequent manual deactivation

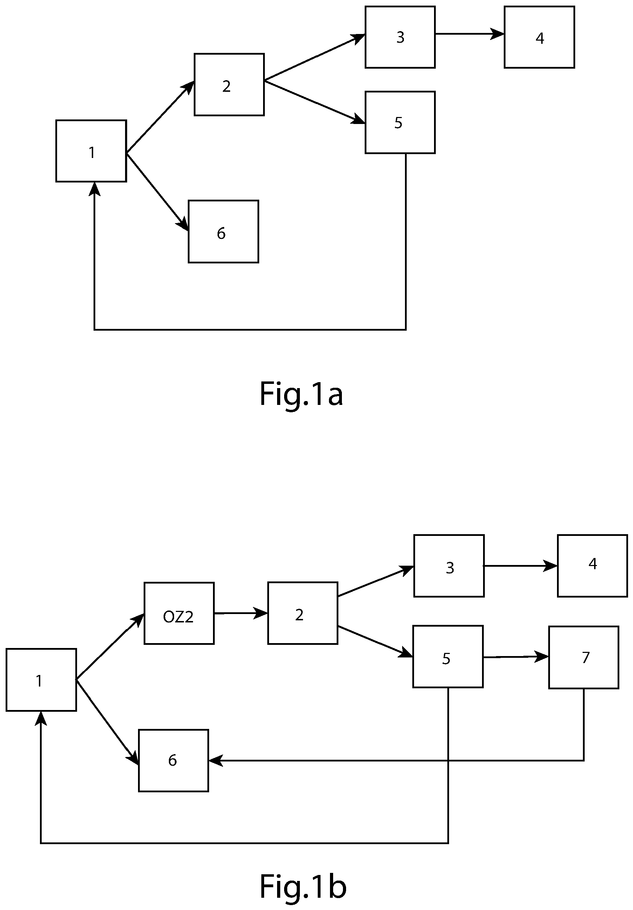

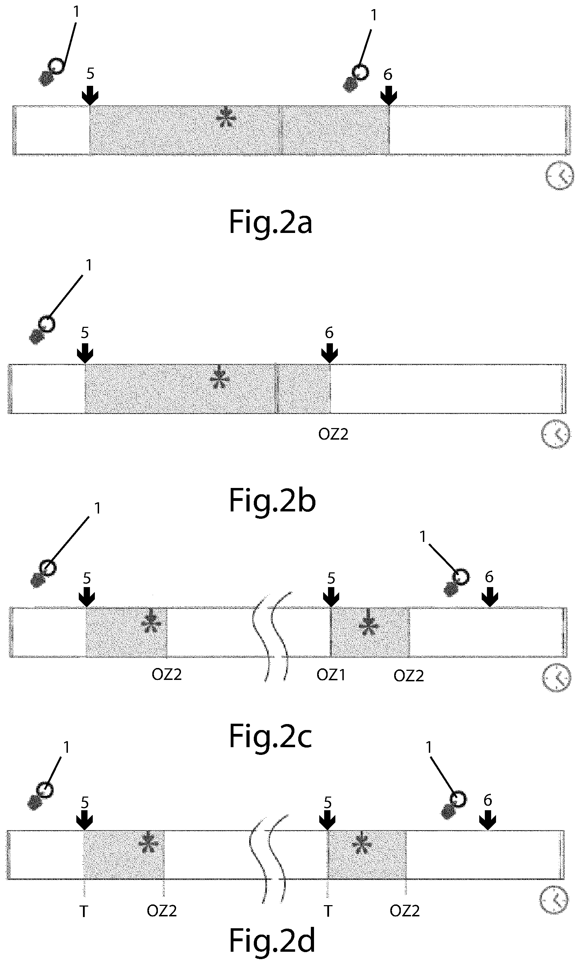

In particular, as shown in the block diagram in Figures 1a and 2a, in this case it is envisaged for the activation to be determined by the interaction of the user with a user interface 1, such as a dedicated actuation button or a selection menu. Subsequently, the selection is sent to a control system 2 which checks if there are any alarms 3 in progress or sensors that would exclude it from starting (such as microcontrollers that control the closing of lids, or alarms connected to a possible lack of power supply, or doors to prevent the leaking of ozone or UVC radiation from the restricted area) so as to block, and therefore not allow, the activation of the sanitizing cycle 4. Alternatively, if there are no alarms 3 in progress, the sanitizing cycle starts 5 and ends only when the user decides to stop it 6 through the user interface 1.

### B) Manual activation with duration defined by a parameter OZ2 (relating to the total duration of the sanitizing cycle, defined by the manual activation of the sanitizing device)

Similarly to the manual activation phase referred to in paragraph A, as shown in detail in Figure 1b and 2b, also in this second case it is the user who starts the sanitizing cycle by means of a user interface 1, also defining a time parameter OZ2 for the total duration of the sanitizing cycle; the selection is sent to a control system 2 which checks if there are any alarms 3 in progress so as to block, and therefore not allow, the activation of the sanitizing cycle 4. Alternatively, if there are no alarms 3 in progress, the sanitizing cycle starts 5 and, starting from the activation of the sanitizing device, an automatic stop 6 of the sanitization itself takes place when a time t = OZ2 elapses (ref. 7 in Figure 1b); naturally, the stop may be manually anticipated at any moment of the sanitizing cycle, as mentioned in paragraph A above.

### C) Automatic periodic timed activation of the sanitizing cycle, defined by a parameter OZ1 (relating to a time interval defined between the various sanitization activations and with a total duration of the sanitizing cycle defined by the aforementioned parameter OZ2)

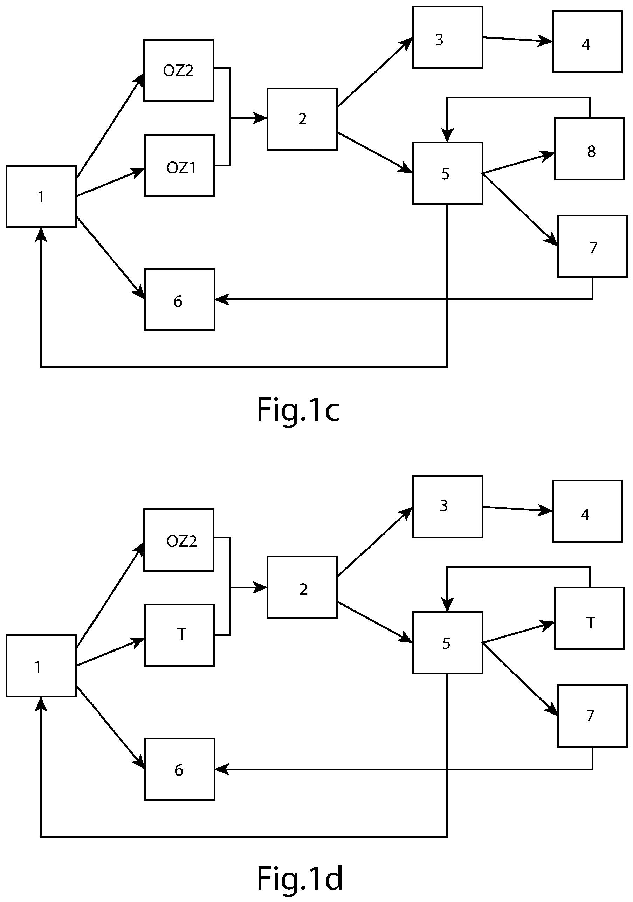

Also in this third case, as shown in the block diagram in Figure 1c and Figure 2c, the user starts the sanitizing cycle through the user interface 1, defining the time parameter OZ2 of total duration of the sanitizing cycle and a further time parameter OZ1 related to the time interval after which the sanitizing cycle is periodically repeated (in the case OZ1 = 0, the case in paragraph B is applicable); similarly to what was said previously, the selection is sent to a control system 2 which checks if there are any alarms 3 in progress so as to block, and therefore not allow, the activation of the sanitizing cycle 4.

Alternatively, if there are no alarms 3 in progress, the sanitizing cycle starts 5 and, starting from the activation of the sanitizing device, it lasts for a time t=OZ2, repeating every t=OZ1, always with a duration equal to the time parameter OZ2.

In this case, the sanitizing device is only stopped manually, as described in paragraph A above; if it is not stopped manually, it continues indefinitely unless there are alarms in progress or there is a power failure.

### D) Scheduled manual activation through the time parameter OZ2

In this fourth case under examination, as shown in the block diagram in Figure 1d and 2d, the sanitizing cycle 5 is started manually through the user interface 1, through which not only is the time parameter OZ2 of the total duration of the sanitizing cycle defined, but also the scheduled repetition of the sanitizing cycles set, thus manually setting the times T in which such sanitizing cycles take place.

Similarly to what has been said previously, the selection made is sent to a control system 2 which checks whether there are any alarms 3 in progress so as to block, and therefore not allow, the activation of the sanitizing cycle 4.

Alternatively, if there are no alarms 3 in progress, the sanitizing cycle starts 5 according to what was defined with the scheduled repetition and, starting from the activation of the sanitizing device, it lasts for a time t=OZ2, repeating every scheduled time T, always with a duration equal to the time parameter OZ2.

In this case too, the sanitizing device is only stopped manually, as described in paragraph C above.

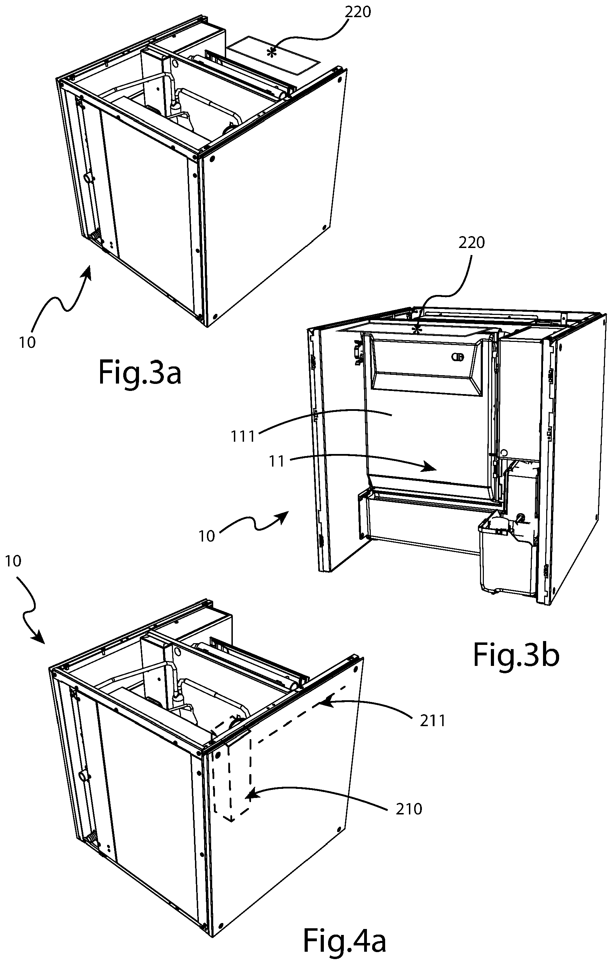

Advantageously, all of the above modes are necessary because the duration and mode of operation depend on many variables:
- different types of sanitizing devices 20 (ozone 210, UVC LED 220, UV lamps 230) and their power;
- environment in which the sanitizing device 20 is installed and impact on users and customers, especially in the case of possible ozone leakage from an ice-making apparatus 10;
- times the apparatus 10 is used, which may have a seasonal nature and therefore a different sanitization frequency or duration;
- different sizes of the apparatus 10 which require lower or higher volume saturations, resulting in necessarily different duration of sanitizing cycles;
- different positioning of the sanitizing devices 20 in the apparatus 10, which causes a different effectiveness of the devices 20, and therefore a different activation timing;
- different temperature and humidity values which, in the case of ozone 210, greatly affect effectiveness, and therefore necessarily require different timing. The above procedure is adaptable to the ice-making apparatus 10 and a first embodiment is shown in Figures 3a, 3b, 4a, 4b and 5, relating to modular makers with a vertical evaporator.

This type of ice-making apparatus 10 comprises a vertical evaporator 11 which is cooled by the cooling circuit. The water descends from the top of the apparatus in the direction of the ground, crossing the vertical alveolar surface of the evaporator 11 so that it freezes, forming ice cubes. Once the desired size of the above cubes is reached, the electronics of the apparatus 10 controls the inversion of the gas cycle, causing the cubes to fall into the ice storage located below the evaporator 11.

In the embodiment under consideration, the sanitizing device 20 is directed and thus acts on the zone of formation of the ice coinciding with the evaporator 11 and on the aforesaid ice deposit. In particular, different types of sanitizing devices can be associated with the aforesaid embodiment.

In detail, as shown in Figures 3a and 3b, if lamps 230 or UVC LEDs 220 are used, they are positioned in the upper region of the apparatus 10 and, advantageously, at the ice-making area, which as previously mentioned, coincides with the evaporator 11. In preferred embodiments, the aforementioned evaporator 11 may be associated with a transparent, UV radiation-resistant deflector 111 in order to make the sanitizing action more effective.

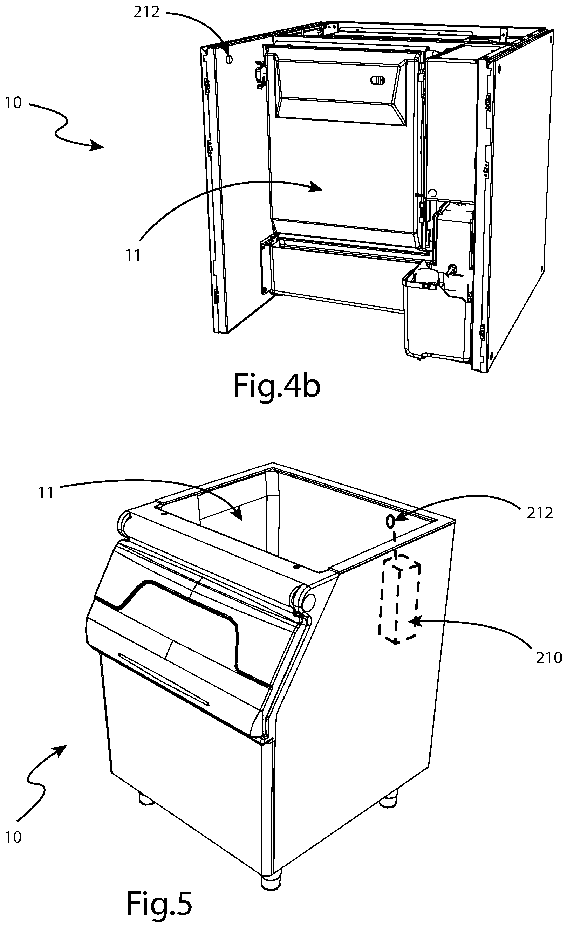

Further, as shown in Figures 4a and 4b, if the sanitizing device 10 envisaged is a gaseous ozone-producing device 210, the device may be positioned either inside or outside the apparatus 10, depending on the overall dimensions, by means of an ozone diffusion outlet 212 placed between the inside and the outside of the apparatus 10 itself.

In detail, the ozone produced is blown into the area of the evaporator 11 by means of a pipe 211 made in particular, of ozone-resistant materials, such as by way of example, silicone; when the ozone-producing device 210 is outside the apparatus 10, the ozone goes from the outside of the apparatus 10 to the inside thereof through the ozone diffusion output 212.

Advantageously, since ozone is heavier than air at the molecular level, once it has been blown at the level of the evaporator 11, due to the effect of gravity it descends into the ice storage 30 below the evaporator or in further embodiments, the ice storage may comprise an external ozone generator, which through a pipe, feeds the ice storage in the ice with sanitizing gas.

Also advantageously, as shown in Figure 5, the ice storage comprises a controllable door in order to obviate the risks of ozone production that could occur if the door of the ice storage were raised.

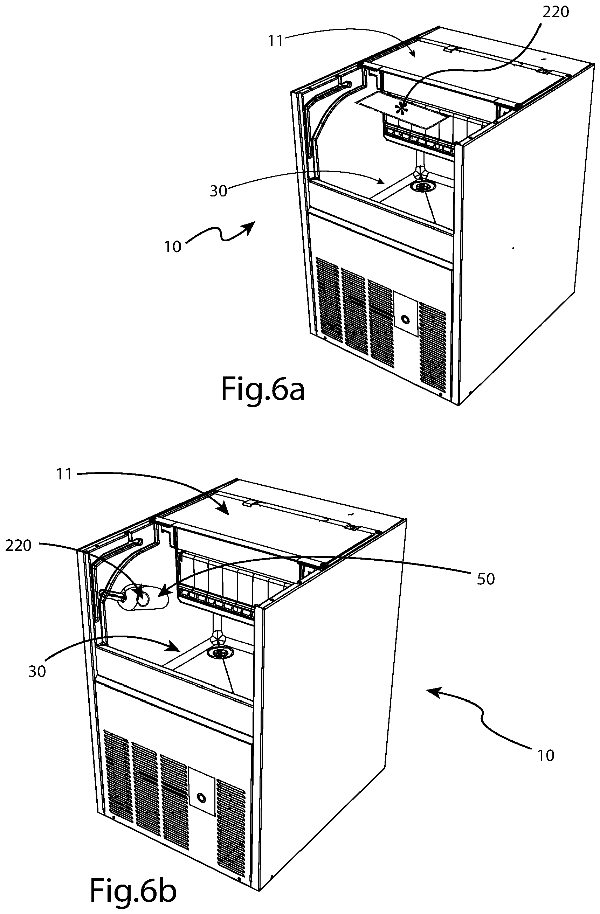

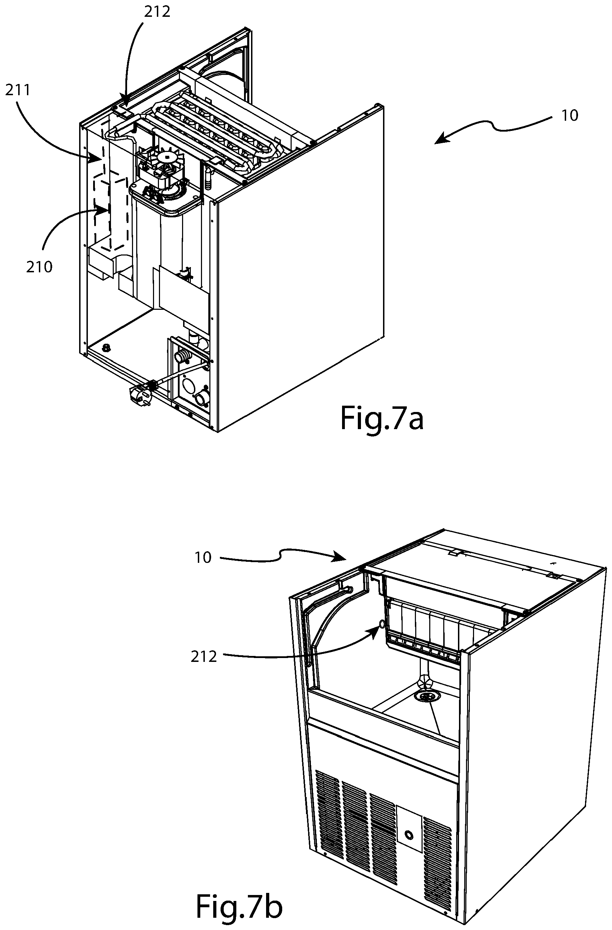

A further embodiment of an apparatus 10 is shown in Figures 6a, 6b, 7a, and 7b relating to spray ice makers.

This type of ice maker has an evaporator 11, located at the top cover of the apparatus 10 and comprising small metal cups, which are chilled by the cooling circuit and face the ground. There is also a pump-driven sprayer below the evaporator, which is adapted to spray water from the bottom upwards onto the chilled cups, freezing them. Once the dimension of the ice cubes reaches the desired size, the electronic board starts the defrosting cycle, which causes the cubes to detach and fall into the ice storage below.

Advantageously, in the case under consideration, sanitization can be directed either to the zone of formation of the ice, which as mentioned above coincides with the evaporator 11, or directly in the ice storage 30 of the apparatus itself.

In detail, as shown in Figure 6a, if lamps 230 or UVC LEDs 220 are used, they are positioned in the upper region of the apparatus 10 and advantageously at the ice-making area, which as previously mentioned, coincides with the evaporator 11.

Moreover, as shown in Figure 6b, a UVC LED plate 220 can be advantageously arranged in the aforesaid apparatus and at the scoop 50 for the removal of ice from the ice storage 30 so as to allow also the sanitization of the inner part of the scoop while it is in the internal stationary position when not in use. Further, as shown in Figures 7a and 7b, if the sanitizing device 10 envisaged is a gaseous ozone-producing device 210, the device may be positioned either inside or outside the apparatus 10, depending on the overall dimensions, by means of an ozone diffusion outlet 212 placed between the inside and the outside of the apparatus 10 itself. Again, the ice storage 30 may have a controllable door to prevent the ozone production from being dispersed outside the apparatus 10.

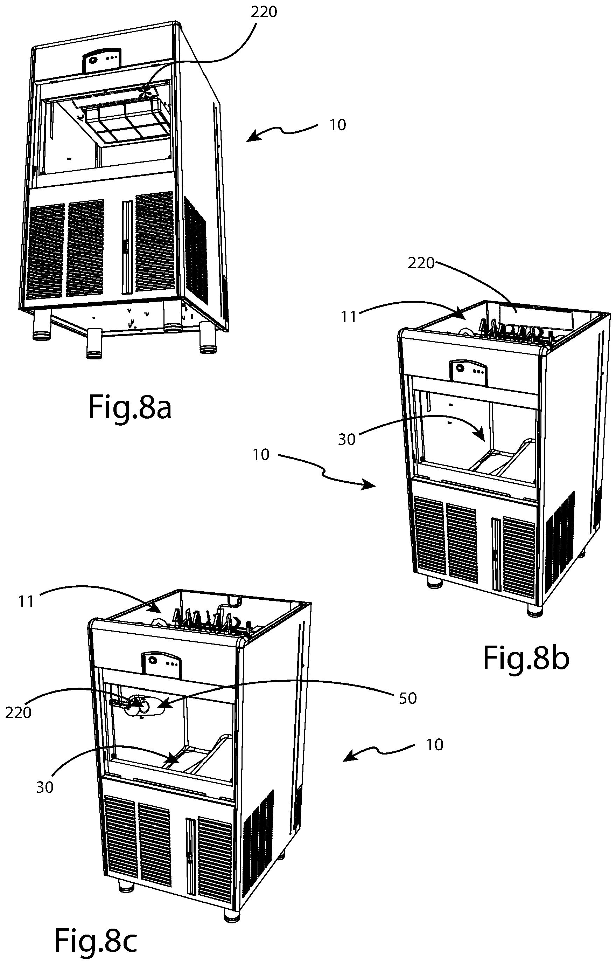

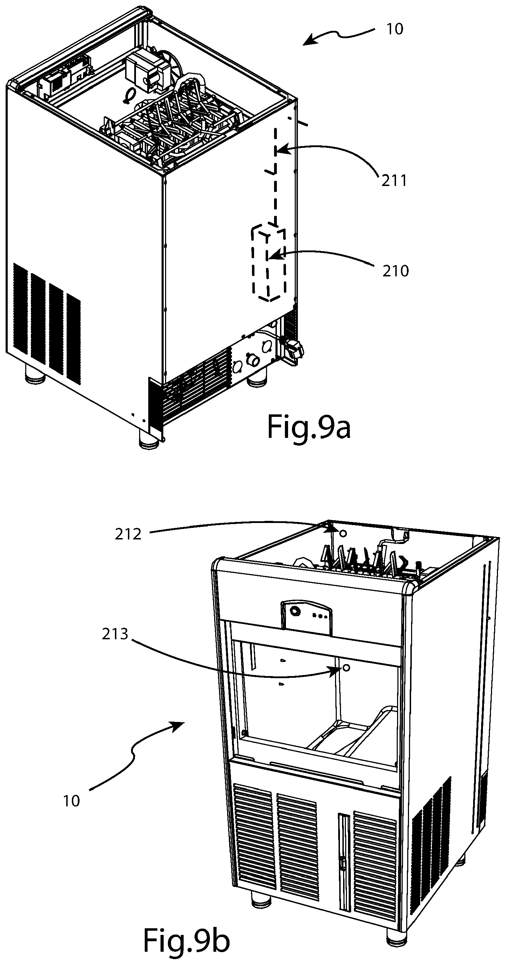

A further embodiment of apparatus 10 is shown in Figures 8a, 8b, 8c, 9a and 9b relating to vane ice makers.

In detail, this type of ice maker comprises an evaporator 11 with attachments made of metal material which are chilled by the cooling circuit. These attachments face the ground and are immersed in a container containing water; the production of ice in this case is determined by the contact of the ice formed with the vanes rotating with the water. As soon as the water is transformed into ice and the desired size of the ice cubes is reached, the electronic board starts the defrosting cycle, which causes the cubes to detach and fall into the ice storage below.

In this case too, sanitization can be directed both on the zone of formation of the ice, which coincides with the evaporator 11, and in the ice storage, which is normally incorporated in the machine and is not separate.

As shown in Figures 8a and 8b, if lamps 230 or UVC LEDs 220 are used, the type of apparatus under consideration may provide for a sanitizing device placed, alternatively or at the same time, in the upper part of the evaporator 11 (Figure 8b) or in the inner and upper part of the apparatus 10 which is intended for the ice storage (Figure 8a).

Moreover, as shown in Figure 8c, a UVC LED plate 220 can be advantageously arranged in the aforesaid apparatus and at the scoop 50 for the removal of ice from the ice storage 30 so as to allow also the sanitization of the inner part of the scoop while it is in the internal stationary position when not in use.

In the event that the sanitizing device is a gaseous ozone dispenser 210, the device may be positioned either inside or outside the apparatus, depending on the overall dimensions, and the ozone is blown through a pipe 211 into the area 212 of the evaporator 11 or of the ice storage or both. Again, the ice storage 30 may include a controllable door to prevent ozone production from being dispersed outside the apparatus 10.

The features of this invention, as the advantages, are apparent from the description provided; finally, it is apparent that this invention is described by way of example only, without limiting the scope of application, according to its preferred embodiments, but it shall be understood that the invention may be modified and/or adapted by experts in the field without thereby departing from the scope of the inventive concept, as defined in the claims herein.

## Claims

1. Ice-making apparatus (10), of the modular type with a vertical spray or vane evaporator comprising:
- an evaporator (11), suitable for being cooled by a cooling circuit and suitable for cooling the water for the formation of ice;
- an ice storage (30) placed in position underlying said evaporator (11);
- a control system (2), suitable to be programmed to cause the fall of said ice formed in said ice storage (30);
- a sanitizing device (20) for the sanitazion of said ice; wherein said sanitizing device (20) acts on a zone of formation of said ice coinciding with said evaporator (11), in correspondence with said ice storage (30) , wherein said sanitizing device (20) is alternatively: a UV lamp (230) or a UVC LED (220), placed above said apparatus (10) and in correspondence of said ice formation zone or located inside said apparatus (10) above said ice storage (30), or
- an ozone-producing device (210) located inside or outside of said apparatus (10) and in that the ozone produced is blown in correspondence of said evaporator (11) through a pipe (211) made of ozone-resistant materials, in particular silicone, **characterized in that** in operation a verification step is provided, by the control system (2) connected to a user interface (1), of the presence of alarms in progress, according to which, if there are alarms in progress, said control system (2) blocks the activation phase of the sanitizing device (20) and, if there are no alarms in progress, said control system (2) starts a sanitizing cycle of said ice making apparatus.

2. Ice-making apparatus (10) as in claim 1, **characterized in that** said UVC LED (220) is placed on said apparatus in correspondence with a scoop (50) for removing the ice from said ice storage (30).

3. Ice-making apparatus as in claim 1, **characterized in that** when said ozone producing device (210) is placed outside said apparatus (10), said pipe (211) passes from outside the apparatus (10) to the inside through an ozone diffusion output (212).

4. Ice-making apparatus (10) as in at least one of claims 1-3, **characterized in that** said ice storage (30) has a controllable door to avoid the loss of ozone in the atmosphere.

5. Control procedure for the sanitization of the ice-making apparatus (10) according to claims 1-4, by means of a sanitizing device (20) of the ozone type (210) and/or UV lamps (230) and/or UVC LED lamps (220), comprising the following steps:
A. activation of said sanitizing device (210, 220, 230) through a user interface (1);
B. verification, by a control system (2) connected to said interface (1), of the presence of alarms (3) in progress, according to which, if there are alarms (3) in progress, said control system (2) blocks said activation phase and, if there are no alarms (3) in progress, said control system starts a sanitizing cycle (5) of said apparatus (10); and
C. Activation (6) of said sanitizing device (20) through said user interface (1) with automatic stop (7) or periodic activation (8) or activation at predetermined instants of said sanitizing device (20), always having the possibility of manual deactivation.

6. Control procedure for the sanitization of ice-making apparatus (10) as in claim 5, **characterized in that** it comprises, before the activation step of the sanitizing device (210, 220, 230), a step for defining a first time parameter (OZ2) relating to the total duration of said sanitization cycle (5) through said user interface (1), said automatic stop (7) being performed when said total duration of the sanitization cycle (5), starting from said activation phase, is equal to said first time parameter (OZ2).

7. Control procedure for the sanitization of ice-making equipment as in the previous claim, **characterized in that** it provides, at the same time as said step of defining the first time parameter (OZ2), a step of defining a second time parameter (OZ1) relating to the interval of time with which said sanitization cycle (5), said sanitization cycle (5) will periodically be repeated having, starting from said activation phase, a duration equal to said first time parameter (OZ2) and periodically repeating each second time parameter (OZ1).

8. Control procedure for the sanitization of equipment as in claim 6, **characterized in that** it provides, at the same time as said step of defining the first time parameter (OZ2), a step of defining a scheduled repetition of said sanitizing cycle (5), through the setting of hourly parameters (T) in which said sanitization cycle takes place with a duration equal to said first time parameter (OZ2).

## Patentansprüche

1. Eismaschine (10), modular aufgebaut, mit vertikalem Spritz- oder Flügelverdampfer, umfassend:
- Einen Verdampfer (11), der zur Kühlung durch einen Kühlkreislauf geeignet ist und zur Kühlung des Wassers geeignet ist, um Eis zu bilden;
- Ein Eislager, (30) welches sich unter dem besagten Verdampfer (11) befindet;
- Ein Steuerungssystem (2), geeignet, so programmiert zu werden, dass das besagte Eis in das besagte Eislager (30) herunterfällt;
- Eine Desinfektionsvorrichtung (20) für die hygienische Reinigung des besagten Eises; wobei besagte Desinfektionsvorrichtung (20) auf eine Zone wirkt, in der die Eisbildung stattfindet und welche mit dem besagten Verdampfer (11) zusammenfällt, in Verbindung mit dem besagten Eislager (30), wobei es sich bei der besagten Desinfektionsvorrichtung (20) alternativ um
- eine UV-Lampe (230) oder eine UVC-LED (220) handelt, die sich über dem Gerät (10) befindet und mit der besagten Eisbildungszone in Verbindung steht oder sich innerhalb des besagten Gerätes (10) oberhalb des besagten Eislagers (30) befindet, oder
- Ein ozonbildendes Gerät (210) innerhalb oder außerhalb der besagten Vorrichtung (10), wobei das erzeugte Ozon in Übereinstimmung mit dem besagten Verdampfer (11) durch ein Rohr (211) geblasen wird, welches aus ozonbeständigem Werkstoff besteht, insbesondere Silikon, **dadurch gekennzeichnet, dass** im Betrieb durch das an eine Benutzeroberfläche (1) angeschlossene Steuersystem (2) das Vorhandensein von Alarmen überprüft wird, wonach bei laufenden Alarmen das besagte Steuersystem (2) die Aktivierungsphase der Reinigungsvorrichtung (20) blockiert und, wenn keine Alarme aktiv sind, das besagte Steuersystem (2) einen Reinigungszyklus der Eismaschine startet.

2. Eisherstellungsgerät (10) nach Anspruch 1, **dadurch gekennzeichnet, dass** die besagte UVC-LED (220) auf das Gerät gelegt wird, in Verbindung mit einer Schütte (50) zur Entfernung des Eises aus dem besagten Eislager (30).

3. Eisherstellungsgerät nach Anspruch 1, **dadurch gekennzeichnet, dass**, wenn die ozonproduzierende Vorrichtung (210) außerhalb des besagten Gerätes (10) platziert wird, das Rohr (211) von außerhalb des besagten Gerätes (10) durch eine Ozondiffusionsabgabe (212) nach innen verläuft.

4. Eisherstellungsgerät (10) nach mindestens einem der Ansprüche 1-3, **dadurch gekennzeichnet, dass** die besagte Eislagerung (30) über eine steuerbare Tür verfügt, um den Verlust von Ozon in der Atmosphäre zu verhindern.

5. Steuerungsverfahren für die Reinigung der Eismaschine (10) nach Anspruch 1-4 mithilfe einer Desinfektionsvorrichtung (20) des Ozon typs (210) und/oder UV-Lampen (230) und/oder UVC-LED-Lampen (220), bestehend aus den folgenden Schritten:
A. Aktivierung des Desinfektionsgeräts (210, 220, 230) durch eine Benutzeroberfläche (1);
B. Überprüfung des Vorhandenseins von Alarmen (3) durch ein an die Schnittstelle (1) angeschlossenes Steuersystem (2), sodass bei vorhandenen Alarmen (3) das besagte Steuersystem (2) die besagte Aktivierungsphase blockiert und, wenn keine Alarme (3) aktiv sind, das besagte Steuerungssystem einen Desinfektionszyklus (5) des besagten Gerätes (10) startet; und
C. Aktivierung (6) der besagten Reinigungsvorrichtung (20) durch die Benutzeroberfläche (1) mit automatischem Stopp (7) oder periodischer Aktivierung (8) oder Aktivierung der besagten Reinigungsvorrichtung (20) bei vorbestimmten Zeitpunkten, wobei immer die Möglichkeit einer manuellen Deaktivierung besteht.

6. Steuerungsverfahren für die Desinfektion von Eisbereitungsgeräten (10) nach Anspruch 5, **dadurch gekennzeichnet, dass** es vor dem Aktivierungsschritt der Desinfektionseinrichtung (210, 220, 230) einen Schritt zur Festlegung eines ersten Parameters (OZ2) umfasst, der sich auf die Gesamtdauer des besagten Reinigungszyklus (5) über die besagte Benutzeroberfläche (1) bezieht, wobei der besagte automatische Stopp (7) durchgeführt wird, wenn die besagte Gesamtdauer des Reinigungszyklus (5) ab der besagten Aktivierungsphase gleich dem besagten ersten Parameter (OZ2) ist.

7. Steuerungsverfahren für die Desinfektion von Geräten zur Eisherstellung nach dem vorhergehenden Anspruch, **dadurch gekennzeichnet, dass** es gleichzeitig mit dem besagten Schritt der Definition des ersten Zeitparameters (OZ2) einen Schritt zur Festlegung eines zweiten Zeitparameters (OZ1) bietet, der sich auf das Zeitintervall bezieht, in dem der besagte Reinigungszyklus (5), besagte Reinigungszyklus (5) periodisch wiederholt wird, wobei dieser ab der besagten Aktivierungsphase eine Dauer aufweist, die dem genannten ersten Zeitparameter (OZ2) entspricht und sich regelmäßig jeden zweiten Zeitparameter (OZ1) wiederholt.

8. Steuerungsverfahren für die Desinfektion von Geräten nach Anspruch 6, **dadurch gekennzeichnet, dass** es gleichzeitig mit dem Schritt der Definition des ersten Zeitparameters (OZ2) einen Schritt zur Definition einer geplanten Wiederholung des besagten Desinfektionszyklus (5) vorsieht, und zwar durch die Einstellung der stündlichen Parameter (T), in denen der besagte Reinigungszyklus mit einer Dauer stattfindet, die dem besagten ersten Zeitparameter (OZ2) entspricht.

## Revendications

1. Appareil de production de glace (10), du type modulaire avec un évaporateur à jet vertical ou à palettes, comprenant:
- un évaporateur (11), apte à être refroidi par un circuit de refroidissement et apte à refroidir l'eau pour la formation de glace;
- un stockage de glace (30) placée en position sous-jacente à l'évaporateur (11);
- un système de contrôle (2), apte à être programmé pour provoquer la chute de ladite glace formée dans ledit stockage de glace (30);
- un dispositif d'assainissement (20) pour l'assainissement de ladite glace; dans lequel ledit dispositif d'assainissement (20) agit sur une zone de formation de ladite glace coïncidant avec ledit évaporateur (11), en correspondance avec ledit stockage de glace (30), dans lequel ledit dispositif d'assainissement (20) est alternativement:
- une lampe UV (230) ou une LED UVC (220), placée au-dessus dudit appareil (10) et en correspondance avec ladite zone de formation de glace ou située à l'intérieur dudit appareil (10) au-dessus dudit stockage de glace (30), ou
- un dispositif de production d'ozone (210) situé à l'intérieur ou à l'extérieur dudit appareil (10) et en ce que l'ozone produit est soufflé en correspondance dudit évaporateur (11) à travers un tuyau (211) en matériaux résistant à l'ozone, en particulier le silicone, **caractérisé en ce qu'**en fonctionnement une étape de vérification est prévue, par le système de contrôle (2) connecté à une interface utilisateur (1), de la présence d'alarmes en cours, selon laquelle, s'il y a des alarmes en cours, ledit système de contrôle (2) bloque la phase d'activation du dispositif d'assainissement (20) et, s'il n'y a pas d'alarmes en cours, ledit système de contrôle (2) démarre un cycle d'assainissement dudit appareil de production de glace.

2. Appareil de production de glace (10) selon la revendication 1, **caractérisé par le fait que** ladite LED UVC (220) est placée sur ledit appareil en correspondance avec une pelle (50) pour retirer la glace dudit stockage de glace (30).

3. Appareil de production de glace selon la revendication 1, **caractérisé par le fait que** lorsque le dispositif de production d'ozone (210) est placé à l'extérieur de l'appareil (10), le tuyau (211) passe de l'extérieur de l'appareil (10) à l'intérieur par une sortie de diffusion d'ozone (212).

4. Appareil de production de glace (10) tel que décrit dans au moins une des revendications 1 à 3, **caractérisé par le fait que** le stockage de glace (30) possède une porte contrôlable afin d'éviter la perte d'ozone dans l'atmosphère.

5. Procédure de contrôle pour l'assainissement de l'appareil de production de glace (10) selon les revendications 1 à 4 au moyen d'un dispositif d'assainissement (20) du type ozone (210) et/ou de lampes UV (230) et/ou de lampes LED UVC (220), comprenant les étapes suivantes:
A. activation dudit dispositif d'assainissement (210, 220, 230) par l'intermédiaire d'une interface utilisateur (1);
B. vérification, par un système de contrôle (2) connecté à ladite interface (1), de la présence d'alarmes (3) en cours, selon laquelle, s'il y a des alarmes (3) en cours, ledit système de contrôle (2) bloque ladite phase d'activation et, s'il n'y a pas d'alarmes (3) en cours, ledit système de contrôle démarre un cycle d'assainissement (5) dudit appareil (10); et
C. activation (6) dudit dispositif d'assainissement (20) via ladite interface utilisateur (1) avec arrêt automatique (7) ou activation périodique (8) ou activation à des instants prédéterminés dudit dispositif d'assainissement (20), avec toujours la possibilité d'une désactivation manuelle.

6. Procédé de contrôle de l'assainissement d'un appareil de production de glace (10) selon la revendication 5, **caractérisé en ce qu'**il comprend, avant l'étape d'activation du dispositif d'assainissement (210, 220, 230), une étape de définition d'un premier paramètre temporel (OZ2) relatif à la durée totale dudit cycle d'assainissement (5) par l'intermédiaire de ladite interface utilisateur (1), ledit arrêt automatique (7) étant réalisé lorsque ladite durée totale du cycle d'assainissement (5), à partir de ladite phase d'activation, est égale audit premier paramètre temporel (OZ2).

7. Procédé de contrôle de l'assainissement d'un appareil de production de glace selon la revendication précédente, **caractérisé en ce qu'**il prévoit, en même temps que ladite étape de définition du premier paramètre temporel (OZ2), une étape de définition d'un deuxième paramètre temporel (OZ1) relatif à l'intervalle de temps avec lequel ledit cycle d'assainissement (5), ledit cycle d'assainissement (5) sera périodiquement répété ayant, à partir de ladite phase d'activation, une durée égale audit premier paramètre temporel (OZ2) et répétant périodiquement chaque deuxième paramètre temporel (OZ1).

8. Procédé de contrôle de l'assainissement d'un appareil selon la revendication 6, **caractérisé en ce qu'**il prévoit, en même temps que ladite étape de définition du premier paramètre temporel (OZ2), une étape de définition d'une répétition programmée dudit cycle d'assainissement (5), par le réglage de paramètres horaires (T) dans lesquels ledit cycle d'assainissement se déroule avec une durée égale audit premier paramètre temporel (OZ2).
